# EUROPÄISCHE PATENTSCHRIFT

(11) **EP 2 591 777 B1**
(45) Veröffentlichungstag und Bekanntmachung des Hinweises auf die Patenterteilung: **14.12.2016**
(21) Anmeldenummer: 12175471.7
(22) Anmeldetag: 09.07.2010
(51) Int. Cl.: A61K 31/198, A61P 11/00, A61P 11/06, A61P 35/00, A61P 43/00

(54) **L-NIL als Inhibitor zur Regeneration der Lunge von an COPD leidenden Patienten**
L-NIL as inhibitor for regenerating the lung of a patient suffering from COPD
L-NIL en tant qu'inhibiteur pour la régénération du poumon chez un patient souffrant de BPCO

(43) Veröffentlichungstag der Anmeldung: 15.05.2013
(62) Teilanmeldung aus: 10169079.0
(73) Patentinhaber: Justus-Liebig-Universität Gießen, 35390 Gießen (DE)
(72) Erfinder: Weissmann, Norbert, 35415 Pohlheim (DE)
(74) Vertreter: Patentanwälte Olbricht Buchhold Keulertz

(56) Entgegenhaltungen:
- HESSLINGER CHRISTIAN ET AL: "Inhibition of inducible nitric oxide synthase in respiratory diseases.", BIOCHEMICAL SOCIETY TRANSACTIONS AUG 2009 LNKD- PUBMED:19614613, Bd. 37, Nr. Pt 4, August 2009 (2009-08), Seiten 886-891, XP008125743, ISSN: 1470-8752
- HESSLINGER C ET AL: "O66. The highly selective iNOS inhibitor BYK402750 exerts potent anti-inflammatory effects in a mouse model of cigarette smoke-induced inflammation", NITRIC OXIDE: BIOLOGY AND CHEMISTRY, ACADEMIC PRESS LNKD- DOI:10.1016/J.NIOX.2008.06.067, Bd. 19, 1. Januar 2008 (2008-01-01), Seite 35, XP023172658, ISSN: 1089-8603 [gefunden am 2008-01-01]
- ORTIZ J L ET AL: "Direct effect of cigarette smoke on human pulmonary artery tension", PULMORNARY PHARMACOLOGY & THERAPEUTICS, ACADEMIC PRESS, GB, Bd. 23, Nr. 3, 1. Juni 2010 (2010-06-01), Seiten 222-228, XP026994300, ISSN: 1094-5539 [gefunden am 2009-11-25]
- V. HAMPL: "Pulmonary vascular iNOS induction participates in the onset of chronic hypoxic pulmonary hypertension", AMERICAN JOURNAL OF PHYSIOLOGY. LUNG CELLULAR AND MOLECULAR PHYSIOLOGY, Bd. 290, Nr. 1, 19. August 2005 (2005-08-19), Seiten 11-20, XP55060352, ISSN: 1040-0605, DOI: 10.1152/ajplung.00023.2005
- MICHAEL SEIMETZ ET AL: "Inducible NOS Inhibition Reverses Tobacco-Smoke-Induced Emphysema and Pulmonary Hypertension in Mice", CELL, CELL PRESS, US, Bd. 147, Nr. 2, 13. August 2011 (2011-08-13), Seiten 293-305, XP028317885, ISSN: 0092-8674, DOI: 10.1016/J.CELL.2011.08.035 [gefunden am 2011-08-29]
- CAROLA SEIFART: "Emerging drugs in chronic obstructive pulmonary disease", EXPERT OPINION ON EMERGING DRUGS, INFORMA HEALTHCARE, UK, vol. 14, 1 January 2009 (2009-01-01), pages 181-194, XP009153211, ISSN: 1472-8214

## Beschreibung

Die vorliegende Erfindung betrifft die Verwendung eines NOS-Inhibitors zur Herstellung eines Mittels zur Regeneration und Heilung der Lunge.

### Beschreibung und Einleitung des allgemeinen Gebietes der Erfindung

Die Aufgabe der Lunge ist es, Blut mit Sauerstoff zu versorgen und gleichzeitig Kohlendioxid zu entsorgen. Es gibt jedoch zahlreiche Lungenerkrankungen, die die Lunge so weit schädigen und zerstören, dass diese Funktionen nicht mehr aufrechterhalten werden können und eine medizinische Behandlung erforderlich wird.

Allerdings können die Lungenschädigungen bei einigen Lungenerkrankungen, wie der chronisch obstruktiven Lungenerkrankung (chronic obstructive pulmonary disease, COPD), Tuberkulose, Lungenemphysem, Lungen- oder Bronchialkarzinom Lungenfibrose und ggf. Asthma so stark sein, dass eine medikamentöse Therapie nicht mehr möglich ist und eine Transplantation nötig wird. Darüber ist bei der Mehrzahl der genannten Lungenerkrankungen höchstens eine Verlangsamung des Voranschreitens der Erkrankung aber keine heilende Therapie möglich.

Für eine Transplantation stehen derzeit viel zu wenige Spenderlungen zur Verfügung, so dass 30 Prozent der Empfänger sterben, bevor sie eine Transplantation bekommen. Darüber hinaus ist die Sterblichkeit im Zuge einer Lungentransplantation hoch.

Seit langem besteht der Wunsch nach einem Mittel zur Regeneration der Lunge, insbesondere nach einer Schädigung oder Zerstörung der Lungenstruktur, was bisher jedoch medizinisch nicht verfügbar ist, da sich die Lunge im Gegensatz zur Haut oder anderen Epithelien nicht spontan regeneriert.

### Stand der Technik

L-NIL [N6-(1-imino-ethyl)-L-Lysin] ist ein wasserlöslicher Wirkstoff und in der Literatur als semi-selektiver iNOS-Inhibitor bekannt (IC₅₀ = 3.3 µM). L-NIL (z.B. als CAS 159190-45-1 oder CAS 150403-89-7) weist folgende Strukturformel auf:

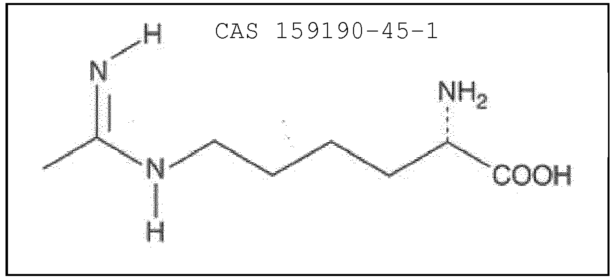

NOS ist das Enzym Stickstoffmonoxid-Synthase, kurz auch NO-Synthase oder NOS genannt. Es katalysiert die Bildung von Stickstoffmonoxid (NO) aus der Aminosäure L-Arginin. Stickstoffmonoxid-Synthase kommt beim Menschen in drei Isoformen (endotheliale eNOS, neuronale nNOS und die induzierbare iNOS) vor, hat aber nur eine kurze Halbwertszeit, so dass es ständig neu gebildet werden muss.

Wissenschaftler, die sich mit chronischen Lungenerkrankungen, wie den Folgen durch Konsum von Tabakrauch, COPD und allergischem Asthma bronchiale befassen, berichten, dass sich einzelne Bereiche der Lunge z.B. das Flimmerepithel selber wieder aufbauen können, wenn z.B. ein Jahr auf den Konsum von Tabak verzichtet wird.
Allerdings sind viele Lungenerkrankungen insbesondere chronisch obstruktive Lungenerkrankungen (chronic obstructive pulmonary disease, COPD), Tuberkulose, Lungenemphysem, Lungen- oder Bronchialkarzinom, Lungenfibrose und Asthma durch eine dauerhafte Entzündung und/oder Umbau (Remodelling) und/oder Zerstörung von strukturbildendem Lungengewebe gekennzeichnet, so dass die Lungenfunktion sehr stark beeinträchtigt ist oder ganz ausfällt. Als Therapie wird in diesen Fällen eine Lungenmaschine eingesetzt und eine Lungentransplantation erforderlich. Eine Regeneration von zerstörtem Lungengewebe (z.B. nach Emphysem) und gleichzeitig eine Wiederherstellung der regelrechten Struktur und Funktion der Lunge ist bisher nicht bekannt oder möglich.

Seifart und Vogelmeier (Seifart, C., Vogelmeier, C. Emerging drugs in chronic obstructive pulmonary disease. Expert Opin. Emerging Drugs 2009, Vol. 14, Seiten 181 bis 194) geben im Zusammenhang mit COPD einen Überblick betreffend therapeutische Strategien und Medikamente, die auf etablierten Konzepten basieren. Dabei gehen die Autoren auf die Behandlung mit Bronchodilatoren und Kortikosteroiden ein. Es besteht die Möglichkeit, Kombinationen verschiedener Bronchodilatoren oder Kombinationen von Bronchodilatoren und Kortikosteroiden einzusetzen, die einen positiven Einfluss auf Parameter wie Exazerbation, Atemnot und Belastungsfähigkeit haben können. Als regenerative Therapien werden der Einsatz von all-trans-Retinsäure und das Einbringen von Stammzellen in die Lunge genannt.

Hesslinger et al. (Hesslinger, C., Strub, A., Boer, R., Ulrich, W.-R., Lehner, M. D., Braun, C. Inhibition of inducible nitric oxide synthase in respiratory diseases. Biochem. Soc. Trans. 2009, Vol. 37, part 4, Seiten 886 bis 891) beschreiben, dass der semi-selektive iNOS-Inhibitor L-NIL in einem elftägigen Zigarettenrauch-Mausmodell, welches bestimmte Aspekte der menschlichen COPD imitierte, den Einstrom von Entzündungszellen und den Zytokinspiegel wirksam minderte. Diese Erkenntnisse wurden durch die Untersuchung von bronchoalveolärer Lavageflüssigkeit (BALF) gewonnen, die im Rahmen einer Bronchoskopie entnommen wurde. Die von Hesslinger et al. erwähnten Reduzierungen des Einstroms von Entzündungszellen und des Zytokinspiegels gehen jedoch nicht mit einer Regeneration der Lunge einher.

### Aufgabe

Aufgabe der vorliegenden Erfindung ist es die Nachteile im Stand der Technik zu überwinden und ein Mittel bereitzustellen, das zur Regeneration von zerstörtem Lungengewebe verwendet werden kann und dabei eine Wiederherstellung der regelrechten Struktur und Funktion der Lunge ermöglicht.

### Lösung der Aufgabe

Diese Aufgabe wird erfindungsgemäß gelöst durch N6-(1-iminoethyl)-L-lysin (L-NIL) zur Verwendung gemäß den Ansprüchen als Inhibitor für die induzierbare NO-Synthase (iNOS) zur Regeneration der Lunge eines Säugetieres, das an einer durch chronische Zigarettenrauch-Inhalation ausgelösten chronischen obstruktiven Lungenerkrankung (COPD) leidet.

Gegenstände, die nicht durch den Schutzumfang der Ansprüche umfasst sind, sind nicht Teil der beanspruchten Erfindung.

Überraschenderweise wurde gefunden, dass Mäuse denen NOS-Inhibitor L-NIL verabreicht wurde eine Regeneration der Lunge aufweisen, wenn diese experimentell durch chronische Zigarettenrauch-Inhalation oder durch andere strukturelle und funktionelle Veränderungen an der Lunge geschädigt wurden. Überraschenderweise wurde damit auch eine Wiederherstellung der regelrechten Struktur und Funktion der Lunge erreicht.

Diese Ergebnisse zeigen dass der NOS-Inhibitor L-NIL (z.B. in Form von CAS 159190-45-1, CAS 150403-89-7) zur Herstellung eines pharmazeutischen Mittels zur Prophylaxe und/oder Behandlung der genannten chronischen Lungenerkrankung und/oder zur Regeneration der Lunge bzw. der Alveolarstruktur geeignet ist.

Insbesondere zeigen die Mäuse, nach oraler Gabe des NOS-Inhibitors L-NIL z.B. im Trinkwasser bei einer Konzentration von 600 µg/ml bereits nach ca. 3 Monaten eine Regeneration der Lunge.

Prinzipiell sind diese Ergebnisse auch auf andere Säugerlungen und insbesondere auf die Lunge von Menschen übertragbar, da diese eine ähnliche Struktur aufweist.

So wird der NOS-Inhibitor L-NIL als erfindungsgemäßes Mittel zur Prophylaxe und/oder Behandlung von chronischen Lungenerkrankungen und/oder zur Regeneration der Lunge bzw. der Alveolarstruktur verwendet.

Dieses erfindungsgemäße den NOS-Inhibitor L-NIL enthaltende Mittel wird insbesondere zur Prophylaxe und/oder Behandlung gegen Erkrankungen der Lunge und der Atemwege bei Säugetieren eingesetzt, die an einem Lungenemphysem und/oder einer pulmonalen Hypertonie leiden.

Das erfindungsgemäße den NOS-Inhibitor L-NIL enthaltende Mittel zur Prophylaxe und/oder Behandlung von chronischen Lungenerkrankungen und/oder zur Regeneration der Lunge bzw. der Alveolarstruktur wird bei Säugern insbesondere Menschen bevorzugt bei Patienten mit chronischen Lungenerkrankungen verwendet.

Damit wird das Mittel in der Human- und Veterinärmedizin verwendet. Das erfindungsgemäße den NOS-Inhibitor L-NIL enthaltende Mittel wird den Patienten, als Teil einer pharmazeutisch akzeptablen Komposition entweder oral, parenteral intravenös / intrararteriell, intramuskulär, subkutan, intrathekal, intracisternal, intraperitoneal, intravasculär, lokal (Puder, Salbe oder Tropfen) oder in Sprayform verabreicht. Die entsprechende Dosierung ist je nach Anwendungsfall für die Therapie vom Arzt festzulegen.

Bevorzugt wird das erfindungsgemäße Mittel oral als wässrige Lösung oder gepresst in Tablettenform verabreicht.

Besonders bevorzugt wird das erfindungsgemäße Mittel mit einem Standardverfahren zum Einbringen von Wirkstoffen in die Lunge verabreicht z.B. als Aerosol. Das erfindungsgemäße Mittel wird als wässrige Lösung bevorzugt mit geeigneten handelsüblichen piezoelektrischen, Düsen-, Ultraschall-Aerosolgeneratoren oder Soft-Mist-Inhalern und Verneblern vernebelt. Beispiele für handelsübliche Vernebler sind: Düsenvernebler wie Bennett-Raindrop®, Pari LC®, Pari LL®, Ventstream®, Ultraschallvernebler wie Multisonic pro®, Pulmosonic®, Systam LS®.

Die Deposition von Aerosolen im Atemtrakt ist abhängig von der Partikelgrößenverteilung des Aerosols. Das erfindungsgemäße Mittel wird bevorzugt in Form von Partikeln mit einem aerodynamischen Massendurchmesser von unter 6 µm eingesetzt, da diese Partikel die Lunge mit Luftröhre, Bronchien und Alveolarbereich zu einem größeren Prozentsatz erreichen, als größere Partikel.

Das erfindungsgemäße Mittel weist eine geeignete Lungengängigkeit auf, was eine Voraussetzung für eine effektive Inhalationstherapie ist.

Für die Anwendung mit Dosieraerosolen oder Pulverinhalatoren kennt der Fachmann zusätzliche Modifikationen der Wirkstoff-Formulierungen z.B. Lösung oder Suspension in Treibmitteln oder die Mikronisierung als Trockenpulver. Beiden Verfahren gemeinsam ist, dass durch Einwirkung mechanischer Energie Aerosoltröpfchen aus dem Flüssigkeitskontinuum herausgelöst werden. Pharmazeutisch akzeptable Kompositionen können die Modifikationen z.B. als Salze, Ester oder Amide beinhalten, sofern sie nach zuverlässiger medizinischer Beurteilung keine übermäßige Toxizität, Irritationen oder allergische Reaktionen am Patienten auslösen.

### Ausführungsbeispiele

### In vivo Studien mit L-NIL in Form eines erfindungsgemäßen Mittels zur Prophylaxe und/oder Behandlung von chronischen Lungenerkrankungen und/ oder zur Regeneration der Lunge bzw. der Alveolarstruktur im Mausmodell

Die Regeneration und Heilung der Lunge durch Gabe des iNOS-Inhibitors L-NIL wird beispielhaft gezeigt in einem Mausmodell der COPD, die durch chronische Zigarettenrauch-Inhalation ausgelöst wird. In diesem Modell werden Mäuse dem Hauptstrom von Zigarettenrauch, in einer Konzentration von 140 mg/m³ für 6 h pro Tag, 5 Tage in der Woche über bis zu 8 Monate ausgesetzt. Es wird gezeigt, dass Mäuse unter diesen Bedingungen innerhalb von 8 Monaten strukturelle und funktionelle Veränderungen der Lunge aufweisen und z.B. eine pulmonale Hypertonie, ein in das Gefäßlumen gerichtetes vaskuläres Remodelling und ein Emphysem entwickeln.

Die durch Rauch verursachten strukturellen und funktionellen Veränderungen der Lunge werden mit geeigneten Techniken quantifiziert. Zur Erfassung des Emphysems wird der "mean linear intercept", die Alveolarseptum-Dicke, und der Luftraum quantifiziert.

Als Parameter zur Erfassung des vaskulären Remodelling werden die Gefäßwandstärke, der Grad der Muskularisierung [nach Doppelfärbung gegen alpha-Aktin der glatten Muskelzellen und von-Willebrand-Faktor (= Endothelzell-Marker)] sowie der Gefäßquerschnitt herangezogen.

Funktionelle Messungen umfassen die Quantifizierung der Lungenfunktion bei spontan atmenden sowie bei künstlich beatmeten Mäusen im Hinblick auf den Atemwegswiderstand, die Compliance und den Volumenfluss. Um funktionelle Veränderungen der Gefäße zu erfassen, werden die Lungen in einem isoliert perfundierten und ventilierten Versuchsaufbau untersucht und die pulmonalvaskulären Widerstände und -Reaktivität gemessen. Die vaskuläre Reaktivität wird so bestimmt, dass die Quantifizierung des Ausmaßes der Vasokonstriktion nach Zugabe von Phenylephrin in Dosis/Wirkungs-Kurven ermöglicht. Im vorkontrahierten Gefäßbett wird die vasodilatorische Kapazität als Antwort auf die Applikation von Acetylcholin und inhaliertem Stickoxid (NO) bestimmt. Dieses Vorgehen erlaubt die Erfassung einer endothelialen (Dys)funktion durch Vergleich des direkten NO-Effekts mit dem durch das Endothel vermittelten.

Durch Rechtsherzkatheterisierung ist es möglich den rechtsventikulären Druck in vivo zu erfassen.

Expressionsmuster der NO Synthase-Isoformen (eNOS, iNOS, nNOS) werden durch quantitative PCR aus Lungenhomogenaten, Lungenwiderstandsgefäßen nach Laser-Mikrodissektion, Alveolarsepten und aus Gefäßen des rechten und linken Herzens und dem systemischen Gefäßbett erstellt.

Auf Proteinebene werden kommerzielle oder spezielle Antikörper zur Analyse eingesetzt.

Die Ergebnisse der molekularen Untersuchungen werden durch Experimente an transgenen Mäusen verifiziert. Dies sind iNOS und eNOS defiziente Mäuse.

Die positive Wirkung von L-NIL auf die Lunge von Mäusen wird parallel auch durch orale Gabe von L-NIL z.B. im Trinkwasser (z.B. in einer Konzentration von 600 µg/ml) gezeigt. In diesen Experimenten zeigt sich ein kompletter Schutz vor der Ausbildung eines Lungenemphysems, wie die Messung über Alveolarmorphometrie zeigt und ein kompletter Schutz vor der Ausbildung von pulmonaler Hypertension, wie die Messung der Hämodynamik, der Herz- und Vaskularmorphometrie zeigt.

Diese Wirkung spiegelt sich wieder bei der Bestimmung der Lungenfunktionsparameter.

Klinisch ist eine Wiederherstellung der strukturellen und funktionellen Beeinträchtigungen der Lunge zu beobachten, die durch 8 monatige Rauchexposition hervorgerufen worden sind.

In einem weiteren Ansatz mit kurativer Behandlung (Beginn der Behandlung mit L-NIL über das Trinkwasser (z.B. in einer Konzentration von 600 µg/ml) erst nach Entstehung des Emphysems und der pulmonalen Hypertonie (8 monatige Rauchexposition) für eine Dauer von 3 Monaten zeigt sich eine komplette Regeneration der Lungenstruktur und der funktionellen und hämodynamischen Parameter, die in Placebo-behandelten Mäusen nicht auftritt.

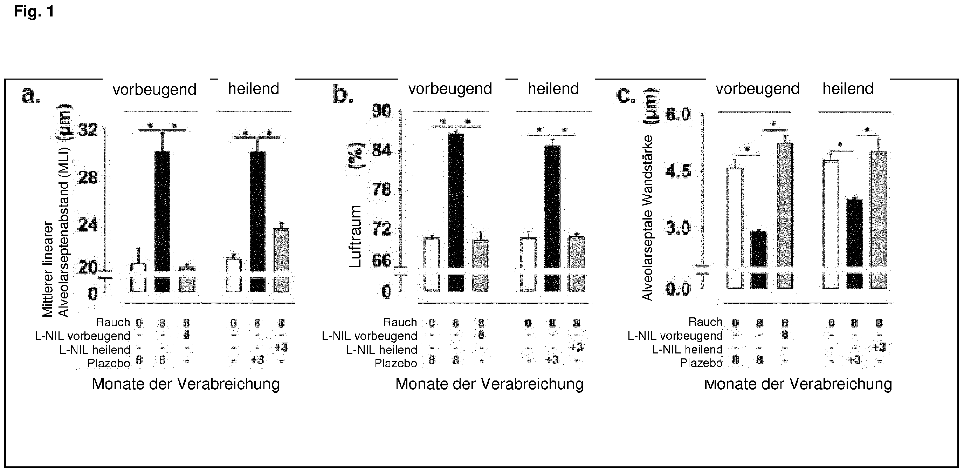

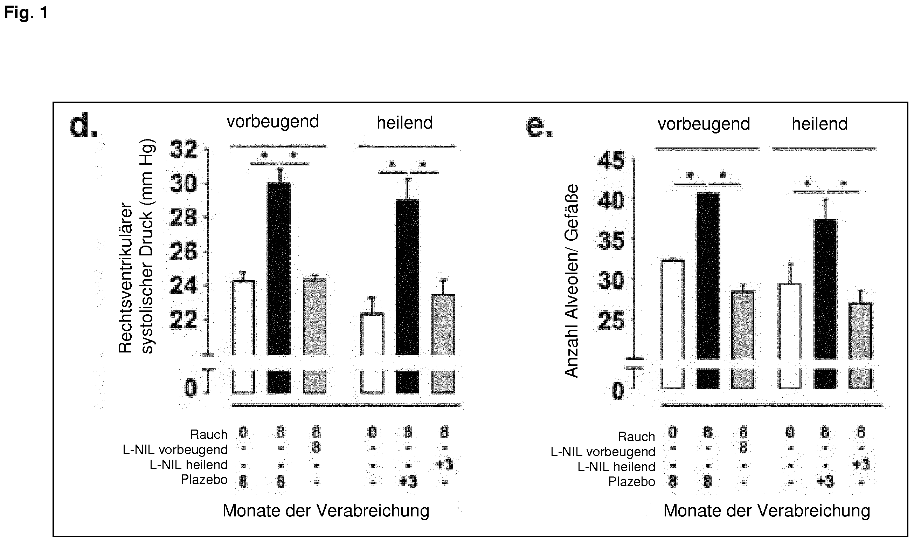

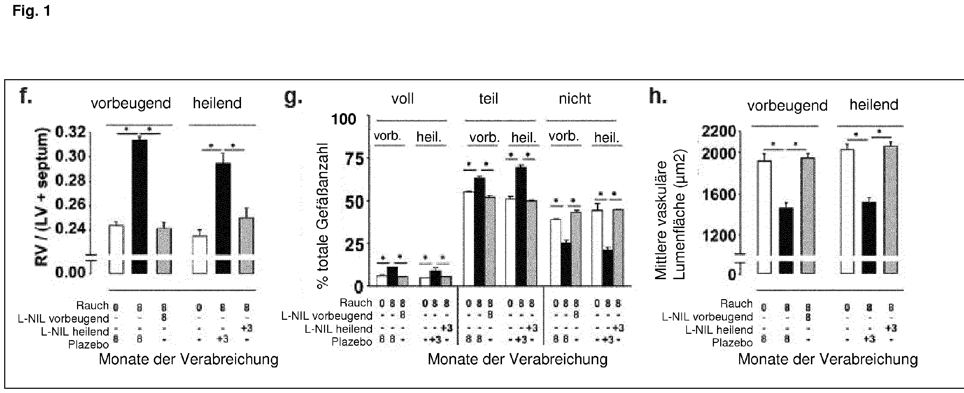

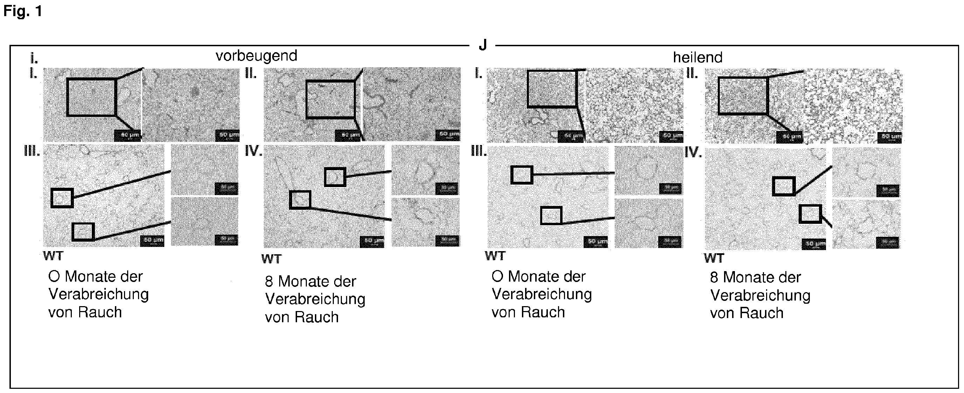

### Figur 1 zeigt die präventive Verhinderung und die kurative Regeneration des Lungenemphysems und der pulmonalen Hypertonie nach Tabakrauchexposition (smoke) durch L-NIL-Behandlung in Wildtyp Mäusen.

Die Behandlung wird entweder von Beginn der Rauchexposition an für 8 Monate (parallel zur Rauchexposition)(vorbeugend, linke Seiten) oder nach Beendigung einer 8 monatigen Rauchexposition (nach Ausprägung des Emphysems) vorgenommen. In diesem zweiten, kurativen Ansatz erfolgt eine 3-monatige Therapie mit L-NIL ohne weitere Rauchexposition (heilend, rechts). Im Vergleich sind Placebo-behandelte Tiere dargestellt.
**(a-c)** Morphometrie der Alveolen **(a)** mean linear intercept (= mittlerer linearer Alveolarseptenabstand), **(b)** air space (=Luftraum, Luftanteil am Lungengewebe), **(c)** septal wall thickness (Alveolarseptale Wandstärke). Quantifiziert von Hematoxylin & Eosin (HE) gefärbten Lungenschnitten. **(d)** Right ventricular systolic pressure (= rechtsventrikulärer systolischer Druck, Rechtsherzdruck) gemessen durch Rechtsherzkatheter in Narkose. **(e)** Verhältnis der Anzahl von Alveolen zu Anzahl der Gefäße quantifiziert an Lungenschnitten nach Färbung für glattmuskuläres alpha-Aktin als Marker für glatte Muskelzellen und von Willebrand Faktor als Marker für Endothelzellen. **(f)** Right heart hypertrophy (=Rechtsherzhypertrophie) als Maß der Masse des rechten Ventrikels (RV) und des linken Ventrikels + Ventrikelseptum (RV/(LV+S)). **(g)** Degree of muscularization (=Muskularisierungsgrad) kleiner pulmonalarterieller Gefäße (Durchmesser 20-70 µm). Die Daten sind als "percentage of total vessel count" (=prozentualer Anteil an der gesamt Gefäßzahl) angegeben für die Kategorien "vollmuskularisert (voll)", "teilmuskularisert (teil)", und "nicht-muskularisiert (nicht)". **(h)** Mean vascular lumen area (mittlere Gefäßfläche, µm²) kleiner pulmonalarterieller Gefäße (Durchmesser 20-70 µm) nach Elastica van Gieson Färbung. **(i)** Representative Histologie aus eNOS^{-/-} Mäusen nach HE Färbung (I, II) oder α-smooth muscle actin (glattmuskuläres alpha-Aktin" und von Willebrand Faktor Färbung (III, IV). **(j)** Representative Histologie aus Lungenschnitten aus iNOS^{-/-} Mäusen nach HE Färbung (I, II) oder Färbung für α-smooth muscle actin und von Willebrand Faktor (III, IV). Jeweils n = 6 Lungen. *signifikante Unterschiede (P<0.05) im Vergleich zu nicht Rauch-exponierten (=gesunde Kontrolle) Tieren (0 Monate).

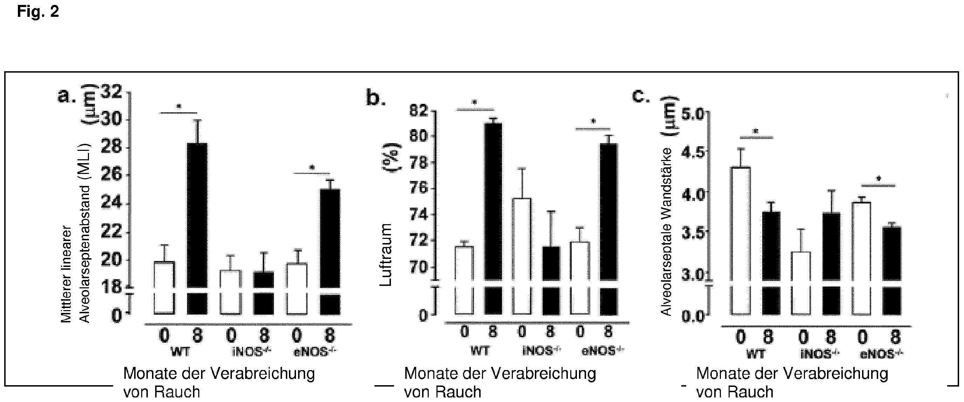

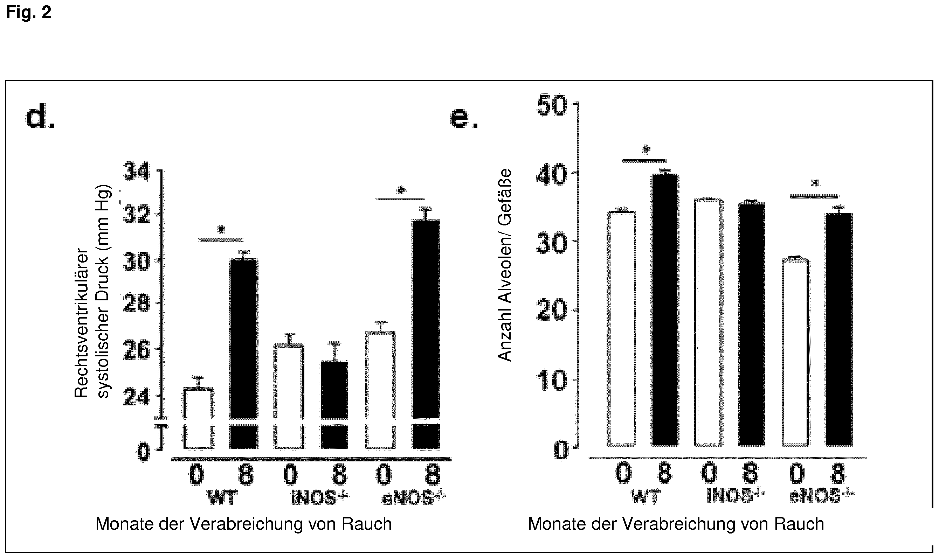

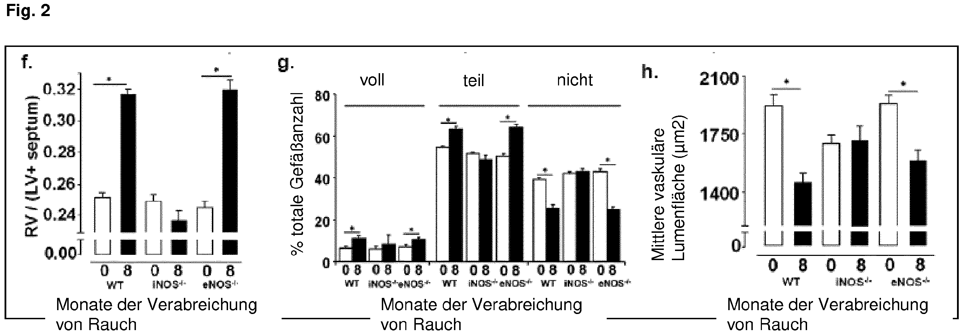

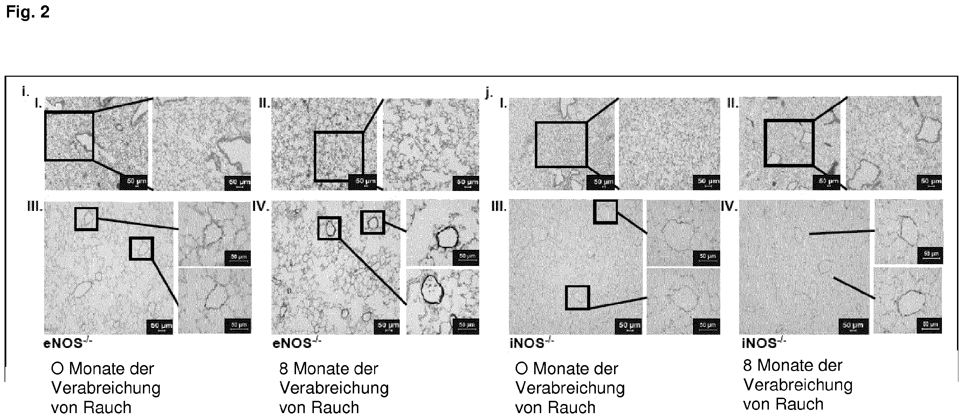

### Figur 2 zeigt einen Vergleich der Emphysementstehung und von pulmonaler Hypertonie induziert durch Tabakrauchexposition für 8 Monate in Wildtyp Mäusen und Mäusen, denen die endotheliale NO Synthase (eNOS) oder induzierbare NO Synthase (iNOS) fehlt.

**(a-c)** Morphometrie der Alveolen **(a)** mean linear intercept (= mittlerer linearer Alveolarseptenabstand), **(b)** air space (=Luftraum, Luftanteil am Lungengewebe), **(c)** septal wall thickness (Alveolarseptale Wandstärke). Quantifiziert aus Hematoxylin & Eosin (HE) gefärbten Lungenschnitten. **(d)** Right ventricular systolic pressure (= rechtsventrikulärer systolischer Druck, Rechtsherzdruck) gemessen durch Rechtsherzkatheter in Narkose. **(e)** (=Verhältnis der Anzahl von Alveolen zu Anzahl der Gefäße) quantifiziert an Lungenschnitten nach Färbung für glattmuskuläres alpha Aktin als Marker für glatte Muskelzellen und von Willebrand factor als Marker für Endothelzellen. **(f)** Right heart hypertrophy (=Rechtsherzhypertrophie) als Maß der Masse des rechten Ventrikels (RV) und des linken Ventrikels + Ventrikelseptum (RV/(LV+S)). **(g)** Degree of muscularization (=Muskularisierungsgrad) kleiner pulmonalarterieller Gefäße (Durchmesser 20-70 µm). Die Daten sind als "percentage of total vessel count" (=prozentualer Anteil an der gesamt Gefäßzahl) angegeben für die Kategorien "vollmuskularisert (voll)" "teilmuskularisert (teil)", und "nicht-muskularisiert (nicht)". **(h)** Mean vascular lumen area (mittlere Gefäßfläche, µm²) kleiner pulmonalarterieller Gefäße (Durchmesser 20-70 µm) nach Elastica van Gieson Färbung. **(i)** Representative Histologie aus eNOS^{-/-} Mäusen, nach HE Färbung (I, II) oder α-smooth muscle actin (glattmuskuläres alpha Aktin) und von Willebrand Faktor Färbung (III, IV). (j) Representative Histologie aus Lungenschnitten aus iNOS^{-/-} Mäusen nach HE Färbung (I, II) oder Färbung für α-smooth muscle actin und von Willebrand Faktor (III, IV). Jeweils n = 6 Lungen. *signifikante Unterschiede (P<0.05) im Vergleich zu nicht Rauchexponierten (=gesunde Kontrolle) Tieren (0 Monate).

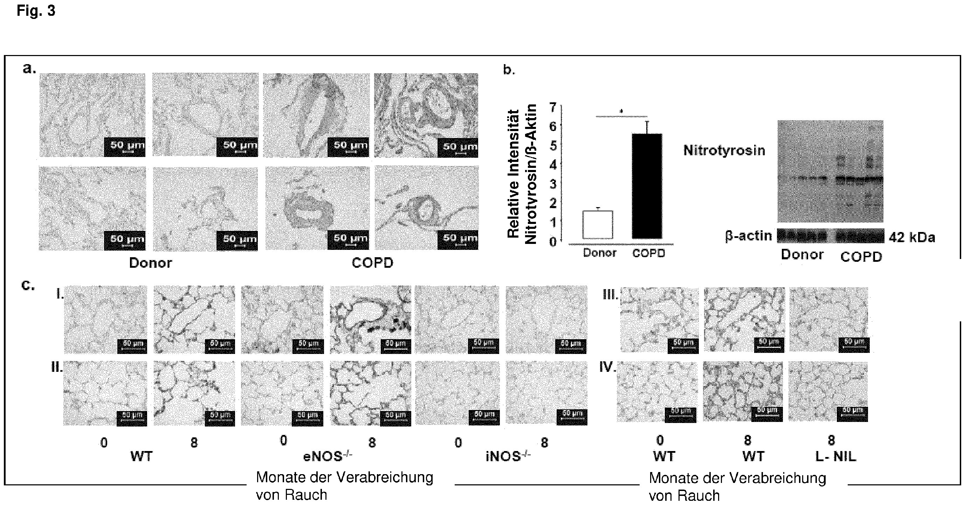

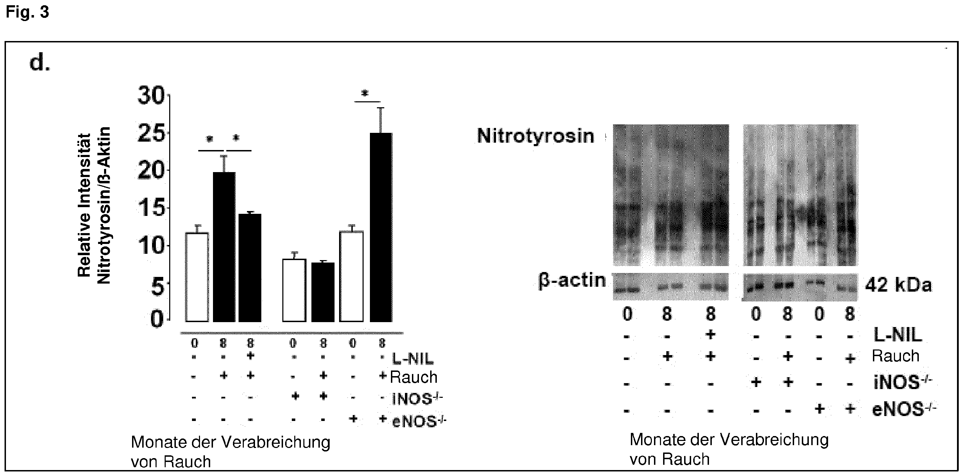

### Figur 3 zeigt die Nitrotyrosin Expression als möglicher Signalweg der L-NIL Therapie. Expression von Nitrotyrosin in menschlichen Lungen von Patienten mit schwerer COPD und gesunden Lungen (Donor) sowie Wildtyp (WT) und eNOS defizienten (eNOS-/-) und iNOS (iNOS-/-) defizienten Mäusen sowie Wildtyp-Mäusen nach L-NIL Behandlung.

**(a)** Lokalisation von Nitrotyrosin und Lungen von GOLD stage IV Patienten und gesunden (Donor) Lungen. Die rote Färbung ist eine Färbung für 3-Nitrotyrosin. In der negativen Kontrolle (negative control) wird kein Primärantikörper verwendet.
**(b)** Western blot für Nitrotyrosin aus Lungenhomogenat von GOLD stage IV Patienten und gesunden Kontrollen (Donor). Daten sind auf die ß-actin Werte normalisiert. Je n=5 Lungen. Densitometrische Daten links, Originalblot rechts.
*signifikante Unterschiede (P<0.05) im Vergleich zu gesunden Kontrollungen. **(c)** Nitrotyrosin-Färbung in Wildtyp (WT), iNOS^{-/-}, eNOS^{-/-},and L-NIL-behandelten Mäusen WT Mäusen (preventive Behandlung). Representative Gefäße (I, III) und Alveolarstruktur (II, IV). Daten sind aus Mäusen, die für 8 Monate Tabakrauchexponiert waren sowie nicht-exponierte, gesunde Kontrollen (0 Monate). **(d)** Western blot für Nitrotyrosin aus Lungenhomogenat in WT, iNOS^{-/-}, eNOS^{-/-} and L-NIL-behandelten WT Mäusen. Data sind normalisiert auf ß-actin und für je n = 5 Lungen dargestellt. *signifikante Unterschiede (P<0.05) im Vergleich zu den jeweiligen nicht-Rauch-exponierten gesunden Kontrollen (0 Monate der Exposition).

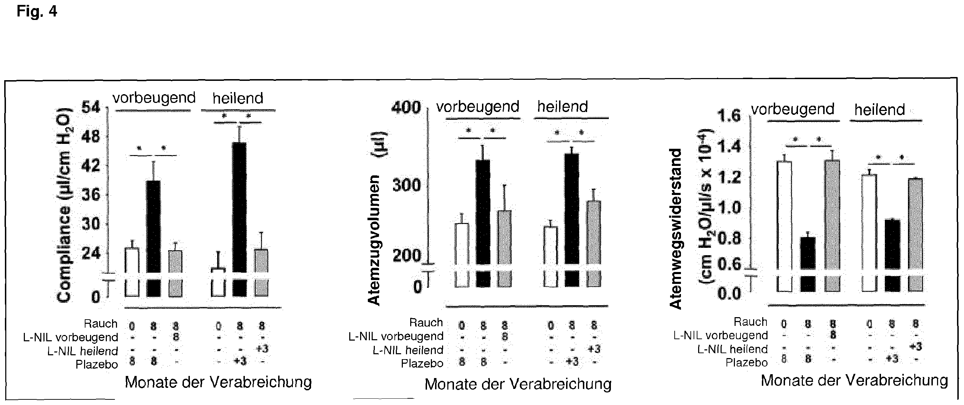

### Figur 4 zeigt Lungencompliance, Atemzugvolumen (=tidal volume) und Atemwegswiderstand (airway resistance) nach 8 monatiger Rauchexposition in Wildtype L-NIL-behandelten Mäusen.

Die Lungenfunktion wird in isoliert perfundierten und ventilierten Lungen nach Explantation gemessen (Negativdruckbeatmung) Ein Emphysem drückt sich in diesem Modell durch einen Abfall des errechneten Atemwegswiderstands aus. Die Behandlung mit L-NIL wird entweder parallel zur Rauchexposition über 8 Monate vorgenommen (vorbeugend, links) oder nach Beendigung der Rauchexposition (8 Monate Rauchexposition) gefolgt von einer 3-monatigen L-NIL oder Placebo-Behandlung. (heilend, rechts). Zum Vergleich sind nicht-Rauchexponierte Tiere (0 Monate) dargestellt. Signifikante Unterschiede (P<0.05) im Vergleich zu Placebo-Behandlung.

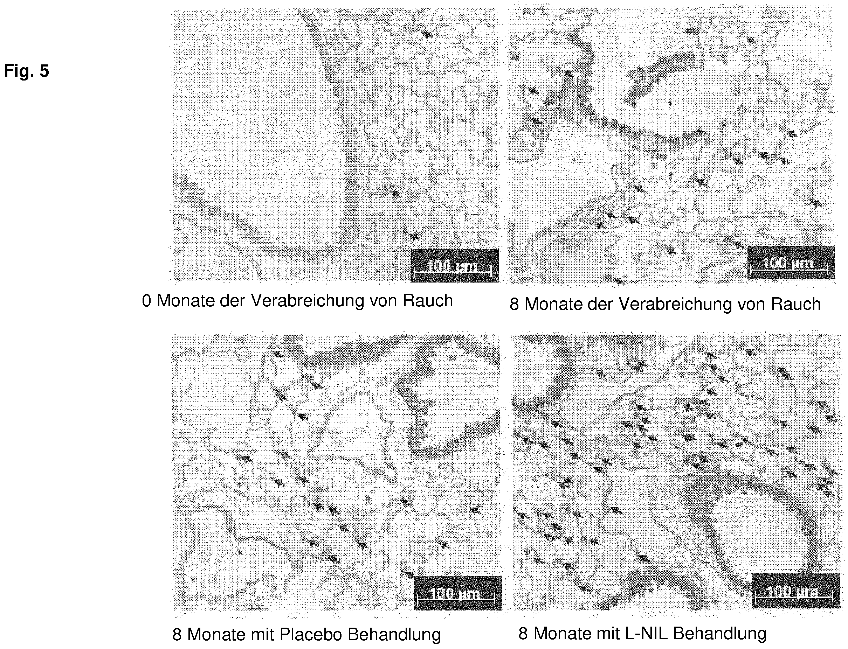

### Figur 5 zeigt die Induktion der Proliferation und der Regeneration des Lungengewebes durch kurative 3-monatige Behandlung mit L-NIL nach vorausgehender Emphyseminduktion durch 8-monatige

**Tabakrauchexposition.** Die Pfeile zeigen Zellen, die positiv für den Proliferationsmarker PCNA sind. Dargestellt sind Lungenausschnitte nicht-raucher Tiere (0 Monate) und Lungen von Tieren die nach 8-monatiger Rauchexposition entweder für 3 Monate mit L-NIL oder mit Placebo behandelt wurden.

## Patentansprüche

1. N6-(1-iminoethyl)-L-lysin (L-NIL) zur Verwendung als Inhibitor für die induzierbare NO-Synthase (iNOS) zur Regeneration der Lunge eines Säugetieres, das an einer durch chronische Zigarettenrauch-Inhalation ausgelösten chronischen obstruktiven Lungenerkrankung (COPD) leidet.

2. L-NIL zur Verwendung als Inhibitor für die iNOS gemäß Anspruch 1, **dadurch gekennzeichnet, dass** die Lunge des Säugetieres durch Zigarettenrauch geschädigt ist.

3. L-NIL zur Verwendung als Inhibitor für die iNOS gemäß Anspruch 1, **dadurch gekennzeichnet, dass** das Säugetier an einem Lungenemphysem und/oder einer pulmonalen Hypertonie leidet.

4. L-NIL zur Verwendung als Inhibitor für die iNOS gemäß einem der vorangegangenen Ansprüche, **dadurch gekennzeichnet, dass** L-NIL als Teil einer pharmazeutisch akzeptablen Komposition oral, inhalativ oder in Tablettenform verabreicht wird.

5. L-NIL zur Verwendung als Inhibitor für die iNOS gemäß Anspruch 4, **dadurch gekennzeichnet, dass** L-NIL oral als wässrige Lösung verabreicht wird.

6. L-NIL zur Verwendung als Inhibitor für die iNOS gemäß Anspruch 4, **dadurch gekennzeichnet, dass** L-NIL als wässrige Lösung als Aerosol verabreicht wird.

7. L-NIL zur Verwendung als Inhibitor für die iNOS gemäß Anspruch 6, **dadurch gekennzeichnet, dass** die Partikel des Aerosols einen aerodynamischen Massendurchmesser von unter 6 µm besitzen.

8. L-NIL zur Verwendung als Inhibitor für die iNOS zur Regeneration der Lunge eines Säugetieres, das an einer durch chronische Zigarettenrauch-Inhalation ausgelösten chronischen obstruktiven Lungenerkrankung (COPD) leidet, wobei L-NIL als Teil einer pharmazeutisch akzeptablen Komposition oral, inhalativ oder in Tablettenform verabreicht wird, **dadurch gekennzeichnet, dass** Salze von L-NIL umfasst sind.

9. L-NIL zur Verwendung als Inhibitor für die iNOS zur Regeneration der Lunge eines Säugetieres, das an einer durch chronische Zigarettenrauch-Inhalation ausgelösten chronischen obstruktiven Lungenerkrankung (COPD) leidet, wobei L-NIL als Teil einer pharmazeutisch akzeptablen Komposition oral, inhalativ oder in Tablettenform verabreicht wird, **dadurch gekennzeichnet, dass** Ester oder Amide von L-NIL umfasst sind.

## Claims

1. N6-(1-iminoethyl)-L-lysine (L-NIL) for use as an inhibitor of inducible NO synthase (iNOS) for regeneration of the lung of a mammal suffering from chronic obstructive pulmonary disease (COPD) triggered by chronic cigarette smoke inhalation.

2. L-NIL for use as an inhibitor of iNOS according to claim 1, **characterised in that** the lungs of the mammal have been damaged by cigarette smoke.

3. L-NIL for use as an inhibitor of iNOS according to claim 1, **characterised in that** the mammal is suffering from emphysema and/or pulmonary hypertension.

4. L-NIL for use as an inhibitor of iNOS according to one of the preceding claims, **characterised in that** L-NIL is administered as part of a pharmaceutically acceptable oral inhalation composition or in tablet form.

5. L-NIL for use as an inhibitor of iNOS according to claim 4, **characterised in that** L-NIL is administered orally as an aqueous solution.

6. L-NIL for use as an inhibitor of iNOS according to claim 4, **characterised in that** L-NIL is administered as an aqueous solution in the form of an aerosol.

7. L-NIL for use as an inhibitor of iNOS according to claim 6, **characterised in that** the particles of the aerosol have a mass median aerodynamic diameter of less than 6 µm.

8. L-NIL for use as an inhibitor of iNOS for regeneration of the lung of a mammal suffering from chronic obstructive pulmonary disease (COPD) triggered by chronic cigarette smoke inhalation, wherein L-NIL is administered as part of a pharmaceutically acceptable oral inhalation composition or in tablet form, **characterised in that** same comprises salts of L-NIL.

9. L-NIL for use as an inhibitor of iNOS for regeneration of the lung of a mammal suffering from chronic obstructive pulmonary disease (COPD) triggered by chronic cigarette smoke inhalation, wherein L-NIL is administered as part of a pharmaceutically acceptable oral inhalation composition or in tablet form, **characterised in that** same comprises L-NIL ester or L-NIL amide.

## Revendications

1. N6-(1-iminoéthyle)-L-lysine (L-NIL) pour une utilisation comme inhibiteur de la NO-synthase inductible (iNOS) en vue de la régénération du poumon d'un mammifère qui souffre d'une maladie pulmonaire obstructive chronique (COPD) déclenchée par l'inhalation chronique de fumée de cigarettes.

2. L-NIL pour une utilisation comme inhibiteur de la iNOS selon la revendication 1, **caractérisée en ce que** le poumon du mammifère est endommagé par de la fumée de cigarettes.

3. L-NIL pour une utilisation comme inhibiteur de la iNOS selon la revendication 1, **caractérisée en ce que** le mammifère souffre d'un emphysème pulmonaire et/ou d'une hypertonie pulmonaire.

4. L-NIL pour une utilisation comme inhibiteur de la iNOS selon l'une des revendications précédentes, **caractérisée en ce que** la L-NIL est administrée par voie orale, par inhalation ou sous forme de comprimés comme partie d'une composition pharmaceutiquement acceptable.

5. L-NIL pour une utilisation comme inhibiteur de la iNOS selon la revendication 4, **caractérisée en ce que** la L-NIL est administrée sous forme de solution aqueuse par voie orale.

6. L-NIL pour une utilisation comme inhibiteur de la iNOS selon la revendication 4, **caractérisée en ce que** la L-NIL est administrée sous forme de solution aqueuse comme aérosol.

7. L-NIL pour une utilisation comme inhibiteur de la iNOS selon la revendication 6, **caractérisée en ce que** les particules de l'aérosol ont un diamètre massique aérodynamique de moins de 6 µm.

8. L-NIL pour une utilisation comme inhibiteur de la iNOS en vue de la régénération du poumon d'un mammifère qui souffre d'une maladie pulmonaire obstructive chronique (COPD) déclenchée par l'inhalation chronique de fumée de cigarettes, la L-NIL étant administrée par voie orale, par inhalation ou sous forme de comprimés comme partie d'une composition pharmaceutiquement acceptable, **caractérisée en ce que** des sels de L-NIL sont compris.

9. L-NIL pour une utilisation comme inhibiteur de la iNOS en vue de la régénération du poumon d'un mammifère qui souffre d'une maladie pulmonaire obstructive chronique (COPD) déclenchée par l'inhalation chronique de fumée de cigarettes, la L-NIL étant administrée par voie orale, par inhalation ou sous forme de comprimés comme partie d'une composition pharmaceutiquement acceptable, **caractérisée en ce que** des esters ou des amides de L-NIL sont compris.
